# EUROPÄISCHE PATENTSCHRIFT

(11) **EP 3 860 464 B1**
(45) Veröffentlichungstag und Bekanntmachung des Hinweises auf die Patenterteilung: **30.07.2025**
(21) Anmeldenummer: 19801686.7
(22) Anmeldetag: 24.10.2019
(51) Int. Cl.: A61B 6/04, A61G 13/08

(54) **PATIENTENLAGERUNGSEINRICHTUNG FÜR EINE RÖNTGENBILDGEBUNGSEINRICHTUNG**
PATIENT POSITIONING DEVICE FOR AN X-RAY IMAGING APPARATUS
DISPOSITIF DE SUPPORT DE PATIENT DESTINÉ À UN DISPOSITIF D'IMAGERIE RADIOGRAPHIQUE

(30) Priorität: 11.12.2018 DE 102018221421
(43) Veröffentlichungstag der Anmeldung: 11.08.2021
(73) Patentinhaber: Siemens Healthineers AG, 91301 Forchheim (DE)
(72) Erfinder: OSWALD, Johannes, 91301 Forchheim (DE); NIEWALDA, Gregor, 91054 Buckenhof (DE); WIETS, Michael, 91094 Langensendelbach (DE)
(74) Vertreter: Siemens Healthineers Patent Attorneys
(86) Internationale Anmeldenummer: PCT/EP2019/078989
(87) Internationale Veröffentlichungsnummer: WO 2020/119992

(56) Entgegenhaltungen:
- CN-A- 106 361 527
- US-A1- 2006 185 090
- US-A1- 2013 133 137
- US-A1- 2016 000 621

## Beschreibung

Die Erfindung betrifft eine Patientenlagerungseinrichtung für eine Röntgenbildgebungseinrichtung, aufweisend eine röntgentransparente Patientenlagerungsplatte zur Lagerung eines Patienten in einer Längsrichtung und zumindest eine die Patientenlagerungsplatte zumindest teilweise tragende Trägereinrichtung.

Variabel anpassbare Patientenlagerungseinrichtungen stellen ein wichtiges Werkzeug bei chirurgischen Eingriffen an Patienten dar. Von zunehmender Wichtigkeit ist zudem die Möglichkeit zur intraoperativen Bildgebung bei derartigen chirurgischen Eingriffen, weshalb bereits Patientenlagerungseinrichtungen vorgeschlagen worden sind, bei denen zumindest die Patientenlagerungsplatte röntgentransparent ist, das bedeutet, die Röntgenstrahlung derart wenig schwächt, dass eine diagnostisch relevante Röntgenbildgebung auch durch die Patientenlagerungsplatte möglich ist.

Problematisch in diesem Zusammenhang ist jedoch, dass eine Vielzahl von chirurgischen Eingriffen bestimmte Stellungen des auf der Patientenlagerungsplatte gelagerten Patienten erfordern. Beispielsweise wird in der Wirbelsäulenchirurgie der Patient typischerweise bäuchlings operiert. Dabei werden als Patientenlagerungseinrichtungen Operationstische, wie beispielsweise der sogenannte Jackson-Table, eingesetzt. Bei einer derartigen Patientenlagerungseinrichtung kann die Patientenlagerungsplatte in der Mitte "aufgestellt" werden, um diverse Patientenpositionen, insbesondere ein Abknicken in der Körpermitte, einstellen zu können. Unterhalb der Patientenlagerungsplatte weist ein derartiger Operationstisch ein Gestänge auf, das eine Röntgendurchleuchtung jedoch extrem erschwert. Ferner sind die versteifenden Elemente aus Metall gefertigt, was die Röntgendurchleuchtung zusätzlich bis hin zur Unmöglichkeit hindert. Dies liegt darin begründet, dass Metalle im Strahlengang starke Artefakte, bis hin zur Unkenntlichkeit des Dargestellten, verursachen.

Über das genannte Beispiel des Abknickens in der Körpermitte hinaus existieren auch andere Aufgabenstellungen bei Operationen, für die Operationstische als Patientenlagerungseinrichtungen vorgeschlagen wurden, bei welchen mehr als eine mittige Verschwenkbarkeit von Teilplatten gegeneinander vorgesehen ist. Beispielsweise ist es bekannt, für beide Beine einzelne Teilplatten vorzusehen, um die sogenannte "Steinschnittlage" zu realisieren. Zwar wurden im Stand der Technik bereits röntgendurchsichtige Gelenke für Operationstische vorgeschlagen, allerdings sind derzeitige, multipel faltbare bzw. verstellbare Operationstische für die gewollten Anwendungen nicht ausreichend oder vollumfänglich röntgendurchsichtig.

Aus der US 2013/133137 A1 und der US 2006/185090 A1 ist je eine Patientenlagerungseinrichtung mit einer röntgentransparenten Lagerungsplatte aus zwei zueinander verschwenkbaren Teilplatten und je einer jeder Teilplatte zugeordneten Trägereinrichtung bekannt, wobei die beiden Trägereinrichtungen miteinander verbunden sind.

Aus der CN 106 361 527 A ist eine Patientenlagerungseinrichtung mit mehreren zueinander verschwenkbaren Teilplatten bekannt.

Aus der US 2016/000621 A1 ist eine Patientenlagerungsvorrichtung aus mehreren zueinander verschwenkbaren Teilplatten bekannt, welche mittels einer Bedieneinheit in Form von z.B. einem Smart Phone oder Tablet ansteuerbar sind.

Der Erfindung liegt daher die Aufgabe zugrunde, eine Patientenlagerungseinrichtung als Operationstisch für chirurgische Eingriffe anzugeben, die zumindest in einem Operationsbereich hervorragende Transparenzeigenschaften für eine Röntgenbildgebung bietet.

Zur Lösung dieser Aufgabe ist erfindungsgemäß eine Patientenlagerungseinrichtung mit den Merkmalen des Anspruchs 1 vorgesehen. Vorteilhafte Ausgestaltungen ergeben sich aus den Unteransprüchen.

Bei einer Patientenlagerungseinrichtung, insbesondere einem Operationstisch, der eingangs genannten Art ist mithin vorgesehen, dass
- die Patientenlagerungsplatte in wenigstens zwei Teilplatten geteilt ist, wobei jede Teilplatte an einer der Teilplatte zugeordneten Trägereinrichtung gelagert ist, und
- jede Trägereinrichtung wenigstens ein Schwenkgelenk zur Verschwenkung der Teilplatten um eine horizontal und senkrecht zur Längsrichtung verlaufende Querachse aufweist.

Dabei kann es sich bei den Trägereinrichtungen insbesondere um Tragesäulen handeln. Nachdem es bei heutigen Patientenlagerungseinrichtungen, insbesondere Operationstischen, üblich ist, eine einzige Trägereinrichtung, insbesondere eine einzige Tragesäule, zu verwenden, schlägt die vorliegende Erfindung mithin vor, nicht nur eine Unterteilung der Patientenlagerungsplatte in Teilplatten vorzunehmen, sondern zusätzlich jeder dieser Teilplatten eine eigene Trägereinrichtung zuzuordnen, an der die entsprechende Teilplatte wenigstens schwenkbar gemäß einer in Querrichtung verlaufenden Querachse gelagert ist. Erfindungsgemäß ist außerdem vorgesehen, dass jede Trägereinrichtung wenigstens eine Höhenverstelleinrichtung zur Höhenverstellung der jeweiligen Teilplatte und eine Längsverstelleinrichtung zur Längsverschiebung der jeweiligen Teilplatte relativ zur Trägereinrichtung aufweist. Dabei sind selbstverständlich auch mehr Freiheitsgrade/Verstelleinrichtungen denkbar, gegebenenfalls auch weitere Schwenkgelenke. Zur Vereinfachung der Beschreibung der erfindungsgemäßen Patientenlagerungseinrichtung seien im Folgenden die Höhenverstelleinrichtungen und/oder Längsverstelleinrichtungen als "Verstelleinrichtungen" zusammengefasst.

Außerdem sind die Trägereinrichtungen erfindungsgemäß mobil ausgebildet.

Letztlich können im Rahmen der vorliegenden Erfindung Trägereinrichtungen mit ihren jeweiligen Teilplatten als eine Art Module der Patientenlagerungseinrichtung, insbesondere des Operationstisches, angesehen werden. Die Kombination von wenigstens zwei, in manchen Fällen zweckmäßigerweise wenigstens drei, Modulen bildet dann die gewünschte Funktion eines vollwertigen Operationstisches, wobei bevorzugt jedes Modul flexibel eingesetzt werden kann. Durch die Positionierung und Einstellung der einzelnen Module unter Nutzung des Schwenkgelenkes sowie gegebenenfalls der Verstelleinrichtungen können verschiedene gängige Operationspositionen realisiert werden. Hierbei wurde erkannt, dass sich der Operationsbereich bzw. Bereich des chirurgischen Eingriffs üblicherweise in dem Bereich befindet, in dem Teilplatten, die gegeneinander verstellt werden können, aufeinandertreffen. Gerade dieser Bereich wird durch die erfindungsgemäße Ausgestaltung bis auf die Teilplatten (und gegebenenfalls Verbindungsmittel oder dergleichen) freigehalten, konkret von Schwenkgelenken, Verstelleinrichtungen und Trägereinrichtungen, so dass dort eine hochqualitative Röntgenbildgebung aufgrund der röntgentransparenten Ausgestaltung der Patientenlagerungsplatte gegeben ist.

Wie bereits erwähnt, können, worauf im Folgenden noch näher eingegangen werden wird, durch Kombination unterschiedlicher Trägereinrichtungen mit Teilplatten unterschiedlich variabel einstellbare Operationstische durch die erfindungsgemäße Patientenlagerungseinrichtung geschaffen werden. Möglich sind spezielle Patientenpositionen für die Urologie, Orthopädie und Gynäkologie, beispielsweise auch die sogenannte Steinschnittlage. In dem speziellen Fall der Steinschnittlage können zwei Module als Beinhalter fungieren, wobei der Vorteil darin liegt, dass ein röntgendurchstrahlbarer Bereich der Patientenlagerungseinrichtung vom Kniegelenk bis zum Brustbereich denkbar wäre, vor allem jedoch intraoperativ, ohne eine Veränderung der Patientenposition zu erfordern. Dabei sei bereits an dieser Stelle angemerkt, dass bei Vorsehen wenigstens einer Längsverstelleinrichtung insbesondere auch ein gleichmäßiges, gemeinsames Verfahren der Teilplatten und somit der gesamten Patientenlagerungsplatte denkbar ist, mithin die Positionierung des Patienten gehalten wird und gleichzeitig eine Umpositionierung eines aufzunehmenden Bereichs derart stattfinden kann, dass störende Trägereinrichtungen sich nicht mehr im Bereich der Röntgenaufnahme mit der Röntgenbildgebungseinrichtung befinden.

Anders ausgedrückt kann im Allgemeinen auch formuliert werden, dass die bisherige zentrale Trägereinrichtung, insbesondere Tragesäule, eines herkömmlichen Operationstisches in wenigstens zwei Trägereinrichtungen, insbesondere Tragesäulen, aufgeteilt wird, so dass eine geschickte Positionierung der Trägereinrichtungen derart vorgenommen werden kann, dass eine Röntgenbildgebung in relevanten Bereichen nicht durch die Trägereinrichtung behindert wird. In besonders vorteilhafter Ausgestaltung kann dabei vorgesehen sein, dass die Trägereinrichtungen in Längsrichtung nach außen angeordnet sind, so dass insbesondere in einer Mitte der Patientenlagerungseinrichtung ein größerer, trägereinrichtungsfreier Raum besteht.

In einer besonders bevorzugten Ausgestaltung der vorliegenden Erfindung ist vorgesehen, dass jede Trägereinrichtung, insbesondere wenigstens ein Fuß der Trägereinrichtung, derart massiv ausgebildet ist, dass bis in beide Endpositionen der Längsverstellung der Schwerpunkt oberhalb des Standbereichs der Trägereinrichtung verbleibt. Massiv bedeutet hierbei, dass Formgebung, Abmessungen und Masse entsprechend gewählt sind. Beispielsweise ist bei größerer Aufstandsfläche des Fußes auf dem Boden bzw. bei weiter auseinanderliegenden Aufstandspunkten eine geringere Masse ausreichend, damit der Schwerpunkt oberhalb des Standbereichs der Trägereinrichtung verbleibt. Mithin können die Trägereinrichtungen, insbesondere deren Module, auch derart stabil ausgeführt sein, dass ihre Funktion, die Teilplatte zu tragen, in jeder zulässigen horizontalen Verschiebungsstellung, insbesondere Längsverschiebungsstellung, gewährleistet ist. Dabei kann insbesondere ausgenutzt werden, dass die Trägereinrichtungen, insbesondere deren Füße, nicht aus röntgentransparentem Material bestehen müssen, mithin entsprechend massiv ausgeführt werden können, um eine ausreichende Stabilität, mithin Standfestigkeit, für alle gewünschten horizontalen Verschiebungen der Teilplatte zu gewährleisten. Es können auch unterschiedliche Trägereinrichtungen für unterschiedliche Einsatzorte vorgesehen werden, beispielsweise weniger massiv dimensionierte Trägereinrichtungen zum Tragen von Extremitäten, massiver dimensionierte Trägereinrichtungen zum Tragen eines Rumpfs oder Korpus. Durch Einsatz von weniger massiv dimensionierten Trägereinrichtungen in Bereichen der Patientenlagerungseinrichtung, in denen diese ausreichend sind, kann in diesen Bereichen Bewegungsfreiraum gewonnen werden.

Vorzugsweise kann die Höhenverstelleinrichtung jeder insbesondere als Tragesäule ausgebildeten Trägereinrichtung als eine Teleskopeinrichtung ausgebildet sein und/oder die Längsverstelleinrichtung wenigstens eine Führungsschiene aufweisen. Eine Teleskopeinrichtung ist hierbei so zu verstehen, dass ein inneres Trägerteil in einem äußeren Trägerteil derart verschiebbar gelagert ist, dass sich die Höhe der Trägereinrichtung durch Verschieben des inneren Trägerteils aus dem äußeren Trägerteil heraus verändern lässt.

Bei einer Ausgestaltung der Trägereinrichtungen als Tragesäulen kann mithin ein insbesondere zylinderförmiger Fuß vorgesehen sein, in dem eine vertikal verstellbare Teleskopsäule (als zweites Trägerteil) verschiebbar gelagert ist. An einem oberen Ende der verschiebbaren Teleskopsäule kann das bevorzugt drehbar gelagerte Schwenkgelenk, also eine insbesondere drehbar gelagerte Gelenkvorrichtung, angebracht sein. In einer konkreten Ausführungsform kann an dem Schwenkgelenk eine in Längsrichtung verschiebbare Schiene befestigt sein, an welcher die Teilplatte angebracht ist. Die Führungsschiene kann beispielsweise in Form einer trapezförmigen Kulisse ausgestaltet sein, wobei selbstverständlich auch andere Ausgestaltungen denkbar sind.

Mit besonderem Vorteil ist jedem Schwenkgelenk und/oder jeder Verstelleinrichtung ein ansteuerbarer Aktor zur jeweiligen Verstellung zugeordnet, wobei die Patientenlagerungseinrichtung ferner eine Steuereinrichtung zur Ansteuerung aller Aktoren aufweist. Mithin ist eine ansteuerbare Aktorik, bevorzugt für alle Verstellmöglichkeiten, vorgesehen, durch die mittels einer Steuereinrichtung, beispielsweise aufgrund von Bedieneingaben und/oder gespeicherten Voreinstellungen, bestimmte Verstellungen der Schwenkgelenke und/oder Verstelleinrichtungen und somit der Teilplatten vorgenommen werden können, um insbesondere vorbestimmte, für chirurgische Eingriffe geeignete Patientenpositionen herstellen zu können. Bevorzugt ist also nicht nur das Schwenkgelenk aktiv steuerbar, sondern auch die Längsverschiebung der Teilplatte, beispielsweise auf der Führungsschiene, und die Höhenverstellung, beispielsweise unter Verwendung einer Teleskopeinrichtung. Die Steuereinrichtung kann insbesondere mehrere Trägereinrichtungen und/oder Module derart ansteuern, dass alle angesteuerten Trägereinrichtungen bzw. Module miteinander koordiniert eine Patientenlagerungseinrichtung in durch die Steuereinrichtung vorgegebener Höhe, Form und Position bilden.

Die Aktoren können dabei auf unterschiedliche Art und Weise realisiert sein, beispielsweise durch einen Seilzug und/oder durch ein Ritzel in Kombination mit einer Zahnstange und/oder durch eine Kette und/oder umfassend einen Motor oder dergleichen. Auch für die Aktoren der Schwenkgelenke sind bereits entsprechende Techniken bekannt.

Zweckmäßige Weiterbildungen der vorliegenden Erfindung können auch vorsehen, dass die Verstelleinrichtungen und/oder Schwenkgelenke und/oder Aktoren wenigstens teilweise ebenso röntgentransparent ausgebildet sind, insbesondere was Komponenten angeht, die in einen möglichen Röntgenbildgebungsbereich einragen können. Beispielsweise können Führungsschienen, Ritzel und/oder Zahnstangen aus röntgentransparentem Material, beispielsweise Kohlefaserwerkstoffen und/oder Kohlefaserverbundwerkstoffen, einfach hergestellt werden.

Eine zweckmäßige Ausgestaltung der vorliegenden Erfindung sieht vor, dass die Steuereinrichtung zur wenigstens teilweisen Kompensation eines durch Verschwenkung der Teilplatten entstehenden Abstands zwischen den Teilplatten durch Längsverschiebung und/oder Höhenverstellung der Teilplatten ausgebildet ist. Hierzu kann die Steuereinrichtung eine über Hardware- und/oder Softwaremittel realisierte Intelligenz aufweisen, die letztlich, insbesondere nach entsprechender bedienerseitiger Anwahl, dafür sorgt, dass die Teilplatten weiterhin eine im Wesentlichen durchgängige Patientenlagerungsplatte bilden. Dabei sei an dieser Stelle noch angemerkt, dass es in diesem Zusammenhang aus Gründen der Stabilität und des Materials zweckmäßig ist, die Teilplatten ausreichend lang zu gestalten und auf eine Ausziehbarkeit oder dergleichen zu verzichten.

Ist also ein Fall gegeben, in dem die Patientenlagerungsplatte mittig in zwei Teilplatten geteilt ist und an den beiden Trägereinrichtungen mit einem entsprechenden Schwenkgelenk angebracht ist und soll eine Abwinkelung des Patienten in der Körpermitte hergestellt werden, können die beiden Teilplatten, eventuell synchron, über die Schwenkgelenke gedreht werden. Dabei beabstanden sich die Teilplatten in der Mitte, was über eine gleichzeitige Schiebebewegung der beiden Teilplatten aufeinander zu kompensiert werden kann.

Auch andere koordinierte Bewegungen sind durch die Steuereinrichtung bzw. eine entsprechende Intelligenz derselben denkbar.

Zweckmäßigerweise kann die Steuereinrichtung zum Empfang von Signalen einer der Patientenlagerungseinrichtung wenigstens zeitweise zugeordneten Bedieneinrichtung ausgebildet sein. Dabei kann die Bedieneinrichtung auch zur Patientenlagerungseinrichtung gehörig sein. Neben der Verwendung von "klassischen" Bedieneinrichtungen, beispielsweise also einer Bedieneinrichtung mit mechanischen Bedienelementen, beispielsweise umfassend Schalter, Knöpfe und dergleichen, sieht eine besonders vorteilhafte Ausgestaltung der vorliegenden Erfindung vor, dass die Bedieneinrichtung ein ein Touchdisplay zur Anzeige einer manipulierbaren Darstellung der Patientenlagerungseinrichtung aufweisendes Mobilgerät, insbesondere ein Tablet, ist. Die manipulierbare Darstellung umfasst dabei zumindest eine Anzeige der aktuellen Positionen und Stellungen der Teilplatten, wobei beispielsweise durch bestimmte bezüglich der angezeigten Teilplatten durchgeführte Gesten deren Position und Stellung (Orientierung) zunächst innerhalb der manipulierbaren Darstellung verändert werden kann. Während dies zum einen unmittelbar zu einer Ansteuerung der Aktoren zum Nachführen der tatsächlichen Position und Stellung führen kann, ist es auch denkbar, zunächst durch Manipulation aller Teilplatten bzw. anderer dargestellter Komponenten der Patientenlagerungseinrichtung eine gewünschte Gesamteinstellung zu definieren, welche dann über ein bestimmtes, insbesondere auch dem Touchscreen zugehöriges Bedienelement anwählbar ist. Nach Anwahl über das entsprechende Bedienelement werden dann durch die Steuereinrichtung die Aktoren angesteuert, um die Gesamteinstellung zu realisieren. Denkbar ist es im Übrigen auch, mehrere Betriebsmodi an der Bedieneinrichtung vorzusehen, beispielsweise einen Direktbedienmodus und einen Gesamteinstellungs-Bedienmodus oder auch Koordinations-Betriebsmodi zur Koordinierten Bewegung der Teilplatten, wie beschrieben. Auch weitergehende Ausgestaltungen, die von modernen Touchscreens bereitgestellt werden, sind im Rahmen der vorliegenden Erfindung selbstverständlich möglich, beispielsweise die Auswahl einzelner Module aus Trägereinrichtung und Teilplatte zur gesonderten Bedienung und dergleichen. Ferner sind auch Ausgestaltungen denkbar, bei denen der Touchscreen einer dauerhaft der Patientenlagerungseinrichtung zugeordneten Bedieneinrichtung zugeordnet ist. Bei einer Ausgestaltung der Bedieneinrichtung als Mobilgerät kann die Bedienfunktionalität über den Touchscreen insbesondere über eine Softwareapplikation ("App") hergestellt werden.

Es sei noch angemerkt, dass die Verbindung der Steuereinrichtung zu der Bedieneinrichtung bevorzugt drahtlos hergestellt werden kann, wobei grundsätzlich bekannte Kommunikationstechnologien, beispielsweise Bluetooth und/oder WLAN, eingesetzt werden können.

Zweckmäßig kann es im Rahmen der vorliegenden Erfindung auch sein, wenn in der Steuereinrichtung unterschiedlichen Operationsarten, mithin Arten des chirurgischen Eingriffs, zugeordnete Grundstellungen gespeichert sind, welche bei benutzerseitiger Auswahl der entsprechenden Grundstellung angefahren werden. Grundstellungen sind mithin Gesamteinstellungen, die bestimmten Arten von chirurgischen Eingriffen zugeordnet sind. Wurde eine derartige Grundstellung angewählt und angefahren, ist es selbstverständlich, insbesondere über die Bedieneinrichtung, welche zweckmäßigerweise auch der Anwahl der Grundstellung dienen kann, möglich, Feineinstellungen auf einen aktuellen Patienten vorzunehmen. Dabei sei darauf hingewiesen, dass auch eine automatische Auswahl einer Grundstellung bzw. eines Vorschlags für eine Grundstellung seitens der Steuereinrichtung ausgegeben werden kann, beispielsweise, wenn die Steuereinrichtung mit einem Informationssystem, beispielsweise einem Krankenhausinformationssystem (KIS) und/oder einem Radiologieinformationssystem (RIS) verbunden ist, aus welchem automatisch ermittelbar ist, welche Art von chirurgischem Eingriff an welchem Patienten durchgeführt werden soll.

In einer besonders vorteilhaften Ausgestaltung der vorliegenden Erfindung kann vorgesehen sein, dass die Steuereinrichtung bei in einer, insbesondere horizontalen, Ebene aneinander anschließenden Teilplatten zur koordinierten Ansteuerung der Verstelleinrichtungen zur gemeinsamen Bewegung der gesamten Patientenlagerungsplatte ausgebildet ist. Soll also beispielsweise ein Patient ohne spezielle Abwinkelungen, beispielsweise also in horizontaler Position, untersucht werden, kann bei einer derartigen Ausgestaltung der Patientenlagerungseinrichtung wie bisher gewohnt vorgegangen werden. Beispielsweise können insgesamte Bewegungen der Patientenlagerungsplatte in der Horizontalen und/oder in der Vertikalen ermöglicht werden. Hierzu bedarf es lediglich der über die Steuereinrichtung hergestellten Synchronisation der beiden Teilplatten bzw. der entsprechenden Verstelleinrichtungen. Es sei bereits an dieser Stelle angemerkt, dass es in diesem Zusammenhang auch denkbar ist, die Teilplatten bei Bedarf zusätzlich mechanisch zu koppeln, womit auch ein eventuell vorhandener schmaler Schlitz zwischen Teilplatten abgedeckt werden kann.

Es sei auch angemerkt, dass die Patientenlagerungseinrichtung, auch wenn sie bevorzugt als ein Operationstisch ausgebildet ist, selbstverständlich auch zweckmäßig für Untersuchungen eines Patienten ohne chirurgischen Eingriff verwendet werden kann, insbesondere dann, wenn bestimmte Stellungen bereits für die Untersuchung gewünscht sind oder Untersuchungsbereiche aufgenommen werden sollen, an deren Stellen sich bei herkömmlichen Patiententischen ggf. eine Trägereinrichtung befindet.

Zweckmäßig kann es im Rahmen der vorliegenden Erfindung ferner sein, wenn die insbesondere röntgenopakes Material umfassenden Schwenkgelenke in Längsrichtung außen in oder an der Trägereinrichtung angeordnet sind. Insbesondere dann, wenn also die Schwenkgelenke röntgenopakes Material umfassen und nicht "im Weg" einer Röntgenbildgebung vorgesehen sein sollen, ist es zweckmäßig, diese möglichst weit außen an oder in der Trägereinrichtung zu verorten, um den mittigen, möglichst von röntgenopakem Material freien Röntgenbildgebungsbereich möglichst groß zu halten.

Wenigstens zwei der wenigstens zwei benachbarten Teilplatten können auf ihrer einander zugewandten Seite durch ein, insbesondere lösbares und/oder flexibles und/oder dehnbares, bevorzugt röntgentransparentes Verbindungsmittel miteinander verbunden sein. Dabei sind im Rahmen der vorliegenden Erfindung verschiedenste Ausgestaltungen denkbar, die je nach hauptsächlichen Anwendungsfällen der Patientenlagerungseinrichtung eingesetzt werden können. So ist es beispielsweise möglich, lösbare Verbindungsmittel zwischen aneinander angrenzenden Teilplatten vorzusehen. Beispielsweise kann durch derartige lösbare Verbindungsmittel eine feste mechanische Kopplung, beispielsweise bei einer gewünschten, festen Stellung der Teilplatten zueinander, herbeigeführt werden, insbesondere bei wie eine gewöhnliche Patientenlagerungsplatte horizontal ausgerichteten und in einer Ebene aneinander anschließenden Teilplatten, wie oben bereits diskutiert wurde. Soll eine Verschwenkung der Teilplatten gegeneinander vorgenommen werden, können die lösbaren Verbindungsmittel dann entfernt werden und es kann die entsprechende Schwenkverstellung erfolgen. Auch eine flexible und/oder dehnbare Kopplung der Teilplatten aneinander kann Vorteile mit sich bringen, wenn ein entsprechendes flexibles und/oder dehnbares Verbindungsmittel vorgesehen ist. Ein derartiges flexibles und/oder dehnbares Verbindungsmittel zwischen Teilplatten kann auch bei Verschwenkungen der Teilplatten gegeneinander und/oder einer sonstigen, zumindest leichten Beabstandung der Teilplatten gegeneinander an den jeweiligen Teilplatten belassen werden. Somit ist insbesondere grundsätzlich eine Abdeckung eines entstehenden Schlitzes gegeben, so dass ein Einklemmen des Patienten vermieden wird.

Denkbar ist im Übrigen ferner auch eine Ausgestaltung, in der eine feste und/oder flexible/dehnbare Kopplung durch die Verbindungsmittel bei Bedarf hergestellt wird, insbesondere, wenn sich die Teilplatten einander bis unterhalb eines Schwellenwerts für den Abstand annähern. Beispielsweise können die Verbindungsmittel in einem solchen Fall magnetische Verbinder aufweisen, die bevorzugt, beispielsweise durch eine entsprechende Oberflächenprofilierung/Führungsstruktur, sich in definierter Weise verbinden und die Kopplung herstellen. Die magnetischen Verbinder können dabei beispielsweise durch elastische Befestigungsmittel an den Seiten der Teilplatten befestigt sein, beispielsweise durch Federn oder dergleichen. Ein insbesondere mittig anzuordnender Gelenkkörper, beispielsweise zylinderförmig und/oder kugelförmig, kann für eine definierte Bewegbarkeit der magnetischen Verbinder gegenüber den Seiten der Teilplatten vorgesehen sein.

In einer zweckmäßigen Weiterbildung kann auch vorgesehen sein, dass für wenigstens zwei der wenigstens zwei benachbarten Teilplatten wenigstens eine der sich zugewandten Oberflächen der Teilplatten und/oder Verbindungsmittel wenigstens einen in eine korrespondierende Führungsvertiefung der anderen Oberfläche eingreifenden, eine Winkelstellung der Teilplatten zueinander bei Eingriff erlaubenden Führungsvorsprung aufweisen. In einem derartigen Fall kann also vorgesehen sein, dass Teilplatten eines Moduls in Längsrichtung so ausgebildet sind, dass eine erste Teilplatte an eine Teilplatte eines angrenzenden Moduls sozusagen angelehnt werden kann, um eine höhere statische Bestimmtheit und somit auch höhere Stabilität zu erreichen. Dabei kann die Verbindung der Teilplatten insbesondere durch eine gegengleiche Ausbildung von Erhöhungen und Aussparungen realisiert werden. In diesem Zusammenhang ist es besonders bevorzugt, wenn der wenigstens eine Führungsvorsprung teilkugelförmig oder teilzylinderförmig ausgebildet ist. Durch die sphärische bzw. kreisförmige Geometrie können die Teilplatten in jedem Anstellwinkel zusammengeführt und gleichzeitig das Abrutschen gegeneinander verhindert werden. Es sei in diesem Zusammenhang angemerkt, dass es auch zweckmäßig sein kann, wenn wenigstens eine der Oberflächen senkrecht zur Längsrichtung und zur Querrichtung dem Führungsvorsprung und/oder der Führungsvertiefung benachbart zurückweichend und/oder Raum für eine Verkippung der Teilplatten gegeneinander bereitstellend ausgebildet ist.

Wie bereits einleitend erläutert, ist es im Rahmen der vorliegenden Erfindung besonders zweckmäßig, wenn die Patientenlagerungsplatte mittig in zwei Teilplatten geteilt ist. Dann kann letztlich ein funktionsgleicher Ersatz für die eingangs genannten Patientenlagerungseinrichtungen, insbesondere Operationstische, nach dem Jackson-Prinzip geschaffen werden.

Alternative Ausgestaltungen zu einer Verwendung von zwei Modulen und einer mittigen Aufteilung der Patientenlagerungsplatte können beispielsweise vorsehen, dass die Patientenlagerungseinrichtung wenigstens drei Teilplatten aufweist und/oder dass wenigstens zwei jeweils einem Bein zugeordnete Teilplatten in Querrichtung aufeinander folgen. Letztere Ausgestaltung ermöglicht es beispielsweise, die sogenannte Steinschnittstellung zu erzielen. Dabei sei angemerkt, dass es insbesondere in diesem Kontext vorteilhaft sein kann, wenigstens für die jeweils einem Bein zugeordneten Teilplatten ein eine Verschwenkung um eine Vertikalachse ermöglichendes Drehgelenk vorzusehen.

Besonders vorteilhaft ist es im Rahmen der vorliegenden Erfindung, wenn die Trägereinrichtungen alle oder gruppenweise baugleich ausgebildet sind, wobei beispielsweise einerseits für höhere Traglast und mechanische Belastung und andererseits für geringere Traglast und mechanische Belastung jeweils baugleiche Trägereinrichtungen vorgesehen sein können. Auf diese Art und Weise ist eine Art Baukastenprinzip realisiert, in dem Teilplatten unterschiedlicher Ausführung, beispielsweise unterschiedlicher Länge und/oder Breite, an Trägereinrichtungen angebracht werden können, wodurch unterschiedliche Konstellationen an Modulen und somit unterschiedliche Patientenlagerungseinrichtungen durch dasselbe Kollektiv aufgebaut werden können. Dies bringt Vorteile bei der Fertigung und Variabilität der Patientenlagerungseinrichtungen mit sich.

Es ist ferner im Rahmen der vorliegenden Erfindung auch denkbar, dass die mobil ausgebildeten Trägereinrichtungen Räder aufweisen. Dies erleichtert nicht nur die Positionierung der Patientenlagerungseinrichtung im Raum, sondern erlaubt es auch, flexibel unterschiedliche Module zu unterschiedlichen konkreten Patientenlagerungseinrichtungen zusammenzustellen. Auch derartigen Rädern können Aktoren, beispielsweise Motoren, zugeordnet sein, die durch die Steuereinrichtung ansteuerbar sein können.

Die Patientenlagerungseinrichtung, insbesondere der Patiententisch, gemäß der vorliegenden Erfindung kann einer speziellen Röntgenbildgebungseinrichtung zugeordnet sein bzw. Teil einer Röntgenbildgebungseinrichtung bilden. Bei der Röntgenbildgebungseinrichtung handelt es sich mit besonderem Vorteil um eine Röntgeneinrichtung mit einem C-Bogen, an dem sich gegenüber ein Röntgenstrahler und ein Röntgendetektor angeordnet sind. Röntgeneinrichtungen mit derartigen C-Bögen haben den Vorteil, dass unterschiedlichste, geeignete Aufnahmegeometrien gewählt werden können und es zudem möglich ist, mit dem C-Bogen zumindest teilweise beispielsweise eine zwischen Trägereinrichtungen bestehende Lücke zu unterfahren und den durch die spezielle Konstruktion der erfindungsgemäßen Patientenlagerungseinrichtung gegebenen Röntgenbildgebungsbereich ideal zu nutzen. Die Steuereinrichtung der Röntgeneinrichtung kann insbesondere zusätzlich auch zur Ansteuerung von Aktoren der Patientenlagerungseinrichtung ausgebildet sein, mithin als Steuereinrichtung der Patientenlagerungseinrichtung dienen. Im Übrigen kann auch eine Bedieneinrichtung der Röntgenbildgebungseinrichtung zusätzlich zur Bedienung der Patientenlagerungseinrichtung, wie beschrieben, vorgesehen sein.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: eine erste Ausgestaltung einer erfindungsgemäßen Patientenlagerungseinrichtung mit drei Modulen,
- Fig. 2: eine zweite Ausgestaltung einer erfindungsgemäßen Patientenlagerungseinrichtung mit zwei Modulen,
- Fig. 3: eine Seitenansicht eines Moduls einer erfindungsgemäßen Patientenlagerungseinrichtung,
- Fig. 4: eine Querschnittsansicht durch das Modul der Fig. 3,
- Fig. 5: eine erste mögliche Gesamteinstellung des ersten Ausführungsbeispiels,
- Fig. 6: eine zweite mögliche Gesamteinstellung des ersten Ausführungsbeispiels,
- Fig. 7: eine dritte mögliche Gesamteinstellung des ersten Ausführungsbeispiels,
- Fig. 8: eine Prinzipskizze eines dritten Ausführungsbeispiels der erfindungsgemäßen Patientenlagerungseinrichtung in einer ersten Gesamteinstellung,
- Fig. 9: eine Prinzipskizze des dritten Ausführungsbeispiels in einer zweiten Gesamteinstellung,
- Fig. 10: eine Aufsicht auf ein viertes Ausführungsbeispiel der erfindungsgemäßen Patientenlagerungseinrichtung,
- Fig. 11: eine fußseitige Ansicht auf das vierte Ausführungsbeispiel,
- Fig. 12: eine erste mögliche Ausgestaltung von Verbindungsmitteln in einem ersten Zustand,
- Fig. 13: die erste mögliche Ausgestaltung der Verbindungsmittel in einem zweiten Zustand,
- Fig. 14: die erste mögliche Ausgestaltung der Verbindungsmittel in einem dritten Zustand,
- Fig. 15: eine zweite mögliche Ausgestaltung von Verbindungsmitteln,
- Fig. 16: eine Ausgestaltung von Oberflächen benachbarter Teilplatten,
- Fig. 17: eine Erläuterung zur Stabilisierung durch die Ausgestaltung der Fig. 16,
- Fig. 18: eine erste mögliche Darstellung auf einem Touchscreen einer Bedieneinrichtung,
- Fig. 19: eine zweite mögliche Darstellung auf dem Touchscreen,
- Fig. 20: eine dritte mögliche Darstellung auf dem Touchscreen, und
- Fig. 21: eine vierte mögliche Darstellung auf dem Touchscreen.

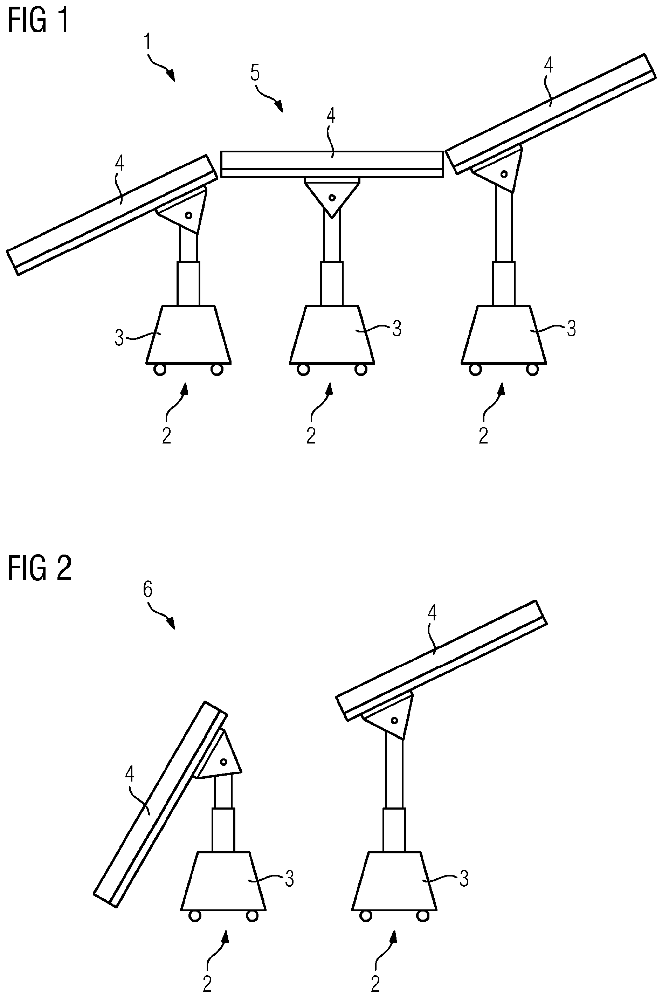

Fig. 1 zeigt eine prinzipielle Ansicht eines ersten Ausführungsbeispiels einer erfindungsgemäßen Patientenlagerungseinrichtung 1, welche vorliegend aus drei Modulen 2 besteht, die jeweils eine Trägereinrichtung 3, hier eine Tragesäule, und eine Teilplatte 4 umfassen. Die Teilplatten 4 ergeben in ihrer Gesamtheit eine Patientenlagerungsplatte 5. Zumindest die Trägereinrichtungen sind alle baugleich.

Fig. 2 zeigt ein weiteres, zweites Ausführungsbeispiel einer erfindungsgemäßen Patientenlagerungseinrichtung 6, welches vorliegend nur zwei Module 2 aufweist, deren Teilplatten 4 die gleiche Größe besitzen, so dass dies mithin als eine mittig aufgeteilte Patientenlagerungsplatte 5 verstanden werden kann. Aufgrund der baugleichen Trägereinrichtungen können beide (und weitere) Ausführungsbeispiele aus dem selben Kollektiv geschaffen werden.

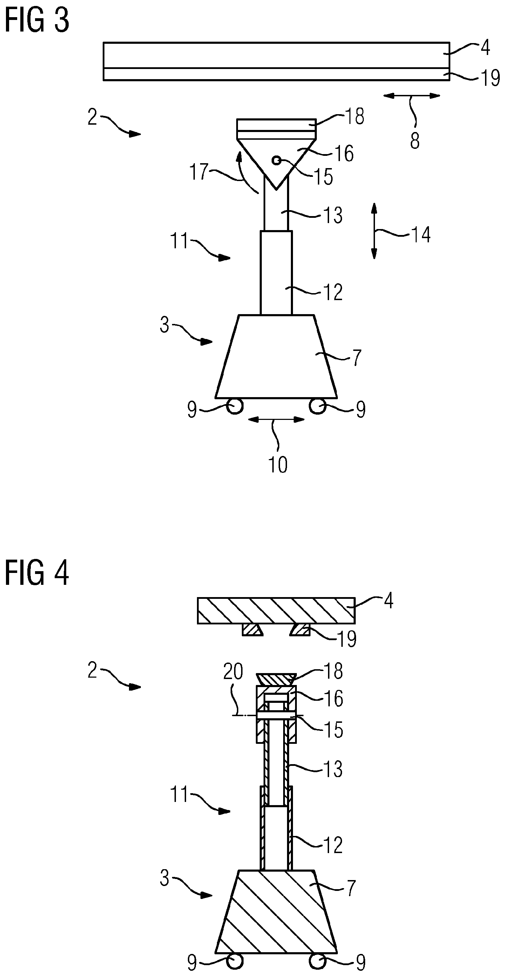

Den Aufbau der Module 2 zeigt Fig. 3 genauer. Dabei sind die Trägereinrichtungen 4, die hier als Tragesäulen ausgebildet sind, für alle Teilplatten 4 aller Ausführungsbeispiele gleich ausgebildet, das bedeutet, beliebige Teilplatten 4 können auf eine Trägereinrichtung 3 aufgesetzt werden, um verschiedene Arten von Patientenlagerungseinrichtungen zu erzeugen. Die Trägereinrichtungen 3 weisen dabei jeweils einen Fuß 7 auf, der derart massiv ausgeführt ist, dass selbst bei in maximaler Längsverschiebung in Längsrichtung 8 der Teilplatte 4 das Modul 2 nicht kippt. Räder 9, die durch entsprechende Arretierungsvorrichtungen arretierbar sind, erlauben die Verfahrbarkeit des Moduls 2, wie durch den Pfeil 10 angedeutet ist. Den Rädern 9 kann ein Aktor, beispielsweise umfassend einen Antriebsmotor, zugeordnet sein, um auch ein automatisches Verfahren, gesteuert durch eine Steuereinrichtung, zu ermöglichen, wie im Folgenden noch näher erläutert werden wird.

Der Fuß 7 trägt eine Teleskopeinrichtung 11, welche eine äußere Teleskopsäule 12 (als erstes Trägerteil) umfasst, in der eine innere Teleskopsäule 13 (als zweites Trägerteil) in Vertikalrichtung 14 verstellbar gelagert ist. Die Verstellung in Horizontalrichtung 14, also die Höhenverstellung, erfolgt ebenso mittels eines hier nicht näher dargestellten Aktors. Die Teleskopeinrichtung 11 wirkt also als eine Höhenverstelleinrichtung.

An die innere Teleskopsäule 13 ist über ein Schwenkgelenk 15 ein weiteres Bauteil 16 befestigt. Das Schwenkgelenk 15 erlaubt, wie durch den Pfeil 17 erläutert wird, eine Verschwenkung um eine zur Horizontalrichtung 14 und zur Längsrichtung 8 senkrechte Querachse in einer senkrecht zur Zeichenebene verlaufenden Querrichtung. Auch das Schwenkgelenk 15 kann über einen ansteuerbaren Aktor betrieben werden.

An dem Bauteil 16 ist eine Führungsschiene 18 als Teil einer Längsverstelleinrichtung angeordnet, die, wie Fig. 4 zu entnehmen ist, im Querschnitt trapezförmig ausgebildet ist und in eine entsprechende Aufnahmeschiene 19 an der Teilplatte 4 eingreift, wobei auch die so gebildete Längsverstelleinrichtung einen hier nicht näher gezeigten, zugeordneten ansteuerbaren Aktor aufweist. Fig. 4 zeigt im Übrigen auch die Querachse 20 des Schwenkgelenks 15.

In anderen Ausgestaltungen können auch weitere Verstellmöglichkeiten/Aktoren realisiert werden. Die Teilplatten 4 sind röntgentransparent ausgebildet, wobei zumindest Teile der Trägereinrichtungen 3 ebenso röntgentransparent realisiert sein können, beispielsweise durch Verwendung von Kohle- und/oder Glasfaserverbundstoffen (beispielsweise GFK, PEEK, ...). Derartige Materialien können auch für die Teilplatten 4 und noch beschriebene optionale Verbindungsmittel verwendet werden.

Die Aktoren der jeweiligen Module 2 werden durch eine gemeinsame, hier nicht näher gezeigte Steuereinrichtung angesteuert, der eine Bedieneinrichtung zumindest zeitweise zugeordnet sein kann. Die Steuereinrichtung und die Bedieneinrichtung können zu der Patientenlagerungseinrichtung 1, 6 selbst gehören, aber auch als Teil einer Röntgenbildgebungseinrichtung, mit der während eines chirurgischen Eingriffs Röntgenbildgebung betrieben werden soll, realisiert sein. Besonders bevorzugt wird, wie im Folgenden noch näher dargelegt werden wird, eine einen Touchscreen aufweisende Bedieneinrichtung verwendet. In der Steuereinrichtung können auch Grundstellungen für verschiedene chirurgische Eingriffe eingespeichert sein, die benutzerseitig aufrufbar sind, woraufhin die Steuereinrichtung die Aktoren zur Herstellung dieser Grundstellungen ansteuert.

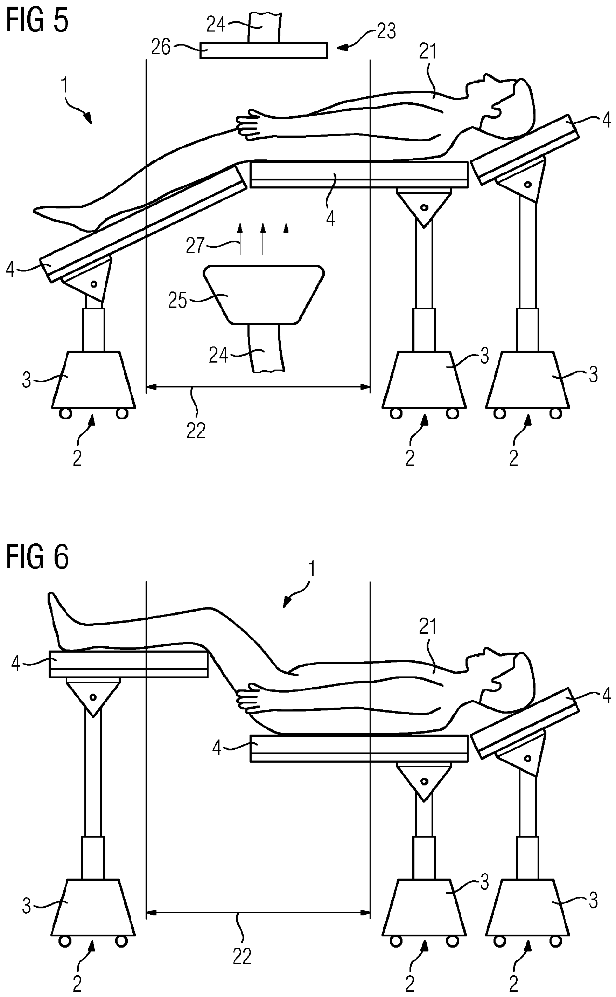

Fig. 5 zeigt am Beispiel der Patientenlagerungseinrichtung 1 eine erste mögliche Gesamteinstellung für einen durchzuführenden chirurgischen Eingriff (oder auch eine sonstige Untersuchung eines Patienten 21). Dabei sind die Teilplatten 4 der äußeren Module 2 ersichtlich mittels der Schwenkgelenke 15 schräggestellt, die mittlere Teilplatte 4 verbleibt eben. Die Trägereinrichtungen 3 des mittleren und des in Fig. 6 linken Moduls 2 sind ersichtlich in Längsrichtung 8 mittels der Längsverstelleinrichtung und/oder der Räder 9 ganz nach außen verfahren, so dass sich ein äußerst großer Röntgenbildgebungsbereich 22 ergibt, der von einer Röntgenbildgebungseinrichtung 23 genutzt werden kann.

Bei der Röntgenbildgebungseinrichtung 23 handelt es sich vorliegend um eine Röntgeneinrichtung mit einem hier nur angedeuteten C-Bogen 24, an dem sich gegenüberliegend ein Röntgenstrahler 25 und ein Röntgendetektor 26 angeordnet sind. Nachdem sich im Wesentlichen lediglich die röntgentransparenten Teilplatten 4 im Röntgenbildgebungsbereich 22 befinden, kann die durch die Pfeile 27 angedeutete Röntgenstrahlung diese problemlos durchdringen und es ist eine hochqualitative Röntgenbildgebung möglich.

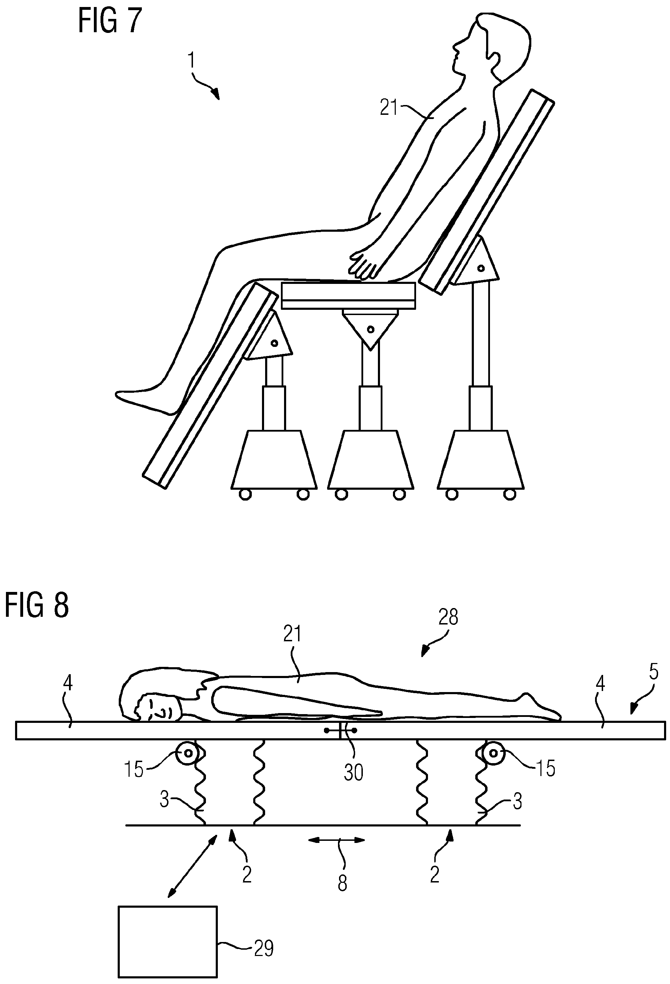

Die Figuren 6 und 7 zeigen weitere Möglichkeiten für Gesamteinstellungen der Patientenlagerungseinrichtung 1. Wie insbesondere aus den Figuren 6 und 7 ersichtlich ist, lassen sich äußerst flexibel Röntgenbildgebungsbereiche 22 an verschiedenen Positionen schaffen.

Fig. 8 zeigt eine Prinzipskizze eines weiteren, dritten Ausführungsbeispiels einer erfindungsgemäßen Patientenlagerungseinrichtung 28. Diese weist in diesem Fall zwei feststehende, als Tragesäulen ausgebildete Trägereinrichtungen 3 auf, an denen die Schwenkgelenke 15 ersichtlich in Längsrichtung 8 nach außen angeordnet sind. Die Patientenlagerungsplatte 5 ist in zwei gleich große, jedoch, wie der Vergleich mit dem Patienten 21 ergibt, verlängerte Teilplatten 4 aufgeteilt. Zudem ist in dieser Prinzipskizze zumindest schematisch auch die Steuereinrichtung 29 zur Steuerung der jeweiligen Aktoren dargestellt.

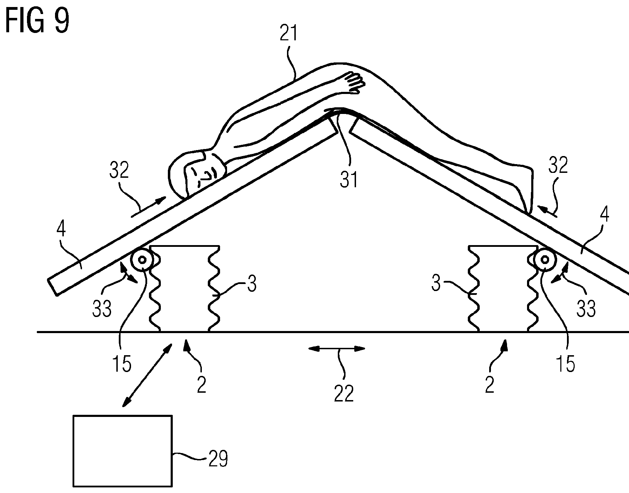

In Fig. 8 liegen beide Teilplatten 4 aneinander anschließend in derselben horizontalen Ebene. Die Patientenlagerungseinrichtung 28 kann hier wie ein normaler Operationstisch verwendet werden, indem die Höhenverstelleinrichtungen und die Längsverstelleinrichtungen der Module 2 synchronisiert betrieben werden, das bedeutet, die Teilplatten 4 bewegen sich wie eine zusammengehörige insgesamte Patientenlagerungsplatte 5. Optionale lösbare Verbindungsmittel 30 können die Teilplatten 4 verbinden und gelöst werden, wenn die Teilplatten 4 beispielsweise mittels der Schwenkgelenke 15 verschwenkt werden sollen, wie dies in Fig. 9 dargestellt ist.

Dort ist das lösbare, als Kopplung wirkende Verbindungsmittel 30 entfernt worden und durch ein flexibles, dehnbares Verbindungsmittel 31 ersetzt worden. Die Schwenkgelenke 15 wurden von der Steuereinrichtung 29 angesteuert, um die Teilplatten 4 jeweils mittig nach oben zu schwenken; gleichzeitig kompensiert die Steuereinrichtung 29 durch Längsverschiebung gemäß dem Pfeil 32 den durch die Verschwenkung, Pfeil 33, entstehenden Abstand. Somit ist wie bei Operationstischen nach dem Jackson-Prinzip eine ideale Position des Patienten 21 für Wirbelsäuleneingriffe möglich, wobei auch eine hervorragende Bildgebung im mittigen Röntgenbildgebungsbereich 22 ermöglicht ist.

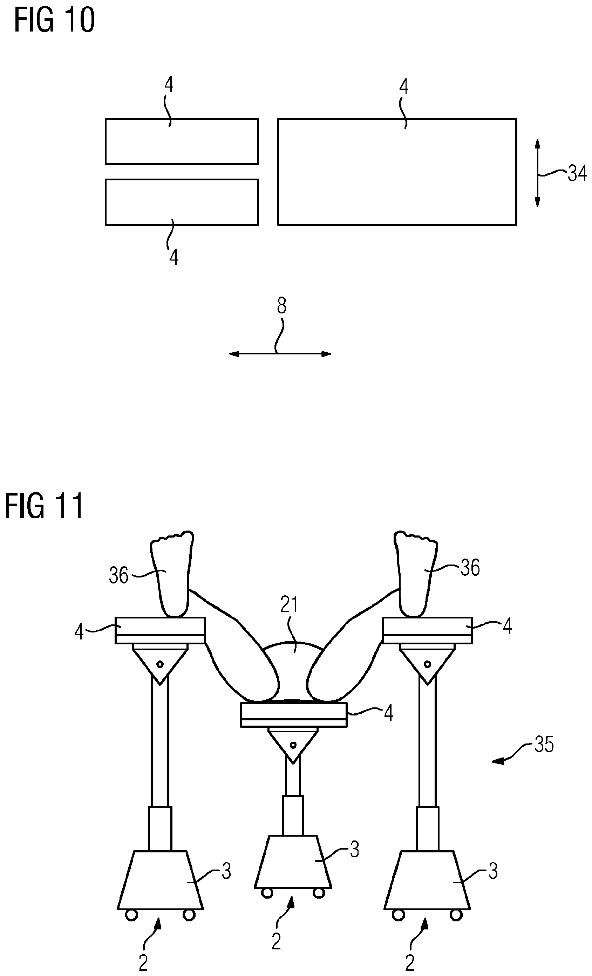

Fig. 10 zeigt eine Aufsicht auf ein weiteres, viertes Ausführungsbeispiel einer erfindungsgemäßen Patientenlagerungseinrichtung 35. Diese umfasst wiederum drei Teilplatten 4, wobei jedoch zwei der Teilplatten 4, die für jeweils ein Bein vorgesehen sind, in Querrichtung 34 aufeinander folgen. Fig. 11 zeigt eine Ansicht der Patientenlagerungseinrichtung 35 von der Fußseite her, wobei die Beine 36 ersichtlich auf den hier höher gestellten Teilplatten 4 der in Querrichtung 34 aufeinander folgenden Module 2 gelagert sind, der Torso des Patienten 21 auf dem in Längsrichtung 8 anschließenden Modul 2. Es lässt sich ersichtlich eine Steinschnittlage erreichen.

Es sei angemerkt, dass bei der Patientenlagerungseinrichtung 35 nach dem vierten Ausführungsbeispiel auch für wenigstens die den Beinen 36 zugeordneten Teilplatten 4 eine seitliche Verschwenkung realisiert werden kann, beispielsweise durch ein entsprechendes Drehgelenk.

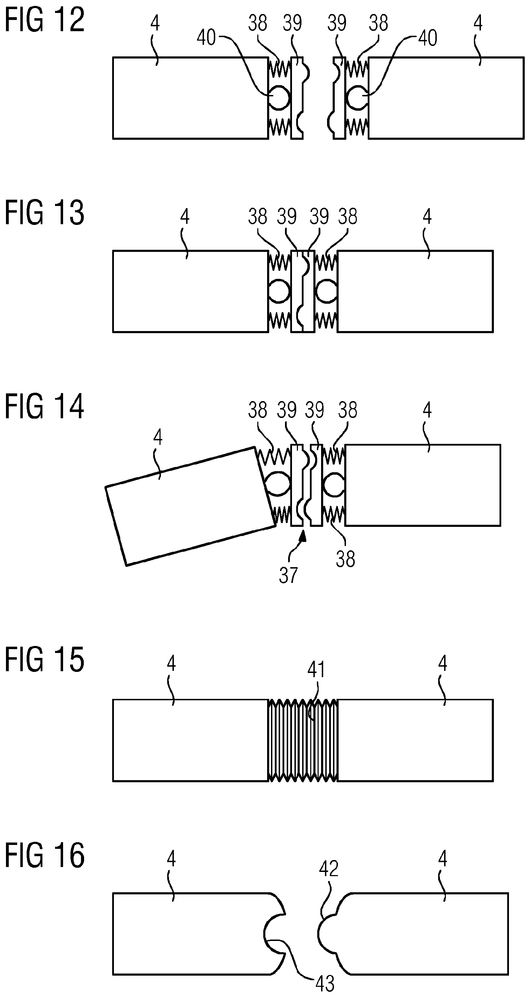

Die Figuren 12 bis 14 zeigen eine Realisierungsmöglichkeit für ein Verbindungsmittel 37 zweier in Längsrichtung benachbarter Teilplatten 4. Ersichtlich ist an den jeweils einander zugewandten Oberflächen der Teilplatten 4 über elastische Befestigungsmittel 38, hier Federn, jeweils ein magnetischer Verbinder 39 befestigt. Nähern sich, wie in Fig. 13 gezeigt, die magnetischen Verbinder 39 einander an, sorgt deren Oberflächenprofilierung dafür, dass sie sich nur auf eine bestimmte Art und Weise miteinander verbinden können, indem Vorsprünge in entsprechende Vertiefungen rutschen. Fig. 14 erläutert zudem, wie eine Verschwenkung der Teilplatten 4 gegeneinander durch die elastischen Befestigungsmittel 38 aufgefangen wird und durch Gelenkkörper 40 stabilisiert und geführt wird, die vorliegend zylindrisch ausgebildet sind.

Fig. 15 zeigt eine weitere Möglichkeit zur Realisierung eines flexiblen und dehnbaren Verbindungsmittels 41 zwischen zwei in Längsrichtung 8 benachbarten Teilplatten 4, welche faltenbalgartig ausgebildet ist und entsprechend dehnbar auseinandergezogen werden kann.

Fig. 16 zeigt eine neben Verbindungsmitteln denkbare Möglichkeit zur gegenseitigen Stabilisierung von in Längsrichtung 8 benachbarten Teilplatten 4, wobei eine der entsprechenden Oberflächen einen Führungsvorsprung 42 aufweist, der in eine Führungsvertiefung 43 der ihr zugewandten Oberfläche der anderen Teilplatte 4 eingreifen kann. Der Führungsvorsprung 42 weist dabei eine teilkreisförmige Querschnittsform auf, ist also insbesondere insgesamt in Querrichtung als ein Teilzylinder ausgebildet. Ferner ist in den Randbereichen Raum für die Verkippung der Teilplatten 4 gegeneinander bereitgestellt.

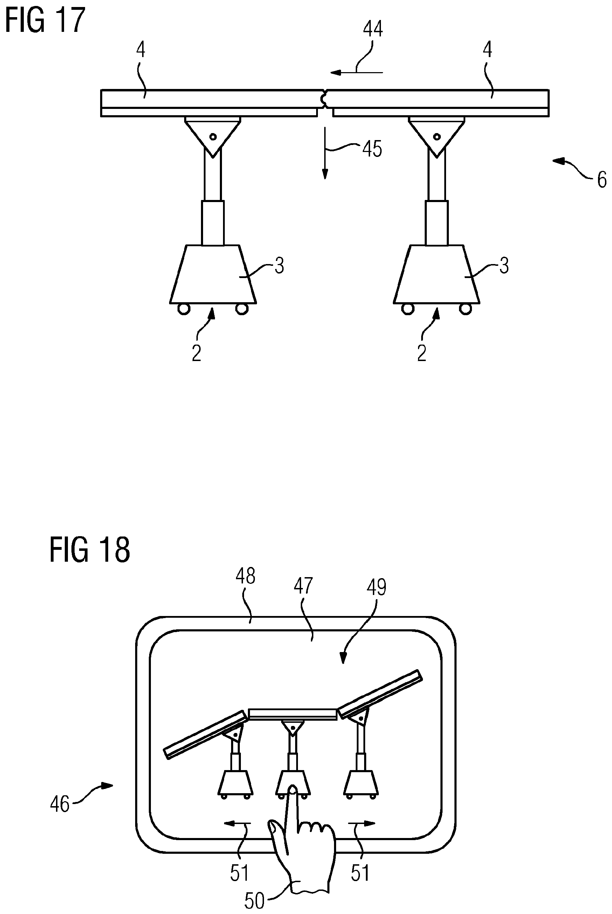

Fig. 17 zeigt, wie der Eingriff der einen Teilplatte 4 in die andere Teilplatte 4 gemäß dem Pfeil 44 gegenüber der Schwerkraft, Pfeil 45, stabilisiert, hier am Beispiel des zweiten Ausführungsbeispiels.

Fig. 18 zeigt schließlich eine Möglichkeit zur Bedienung der Patientenlagerungseinrichtung 1, 6, 28, 35 mittels einer Bedieneinrichtung 46 mit einem Touchscreen 47, hier einem Tablet 48. Das Tablet 48 kann dabei über eine drahtlose Kommunikationsverbindung mit der Steuereinrichtung 29 verbunden sein.

Gemäß Fig. 18 ist eine Darstellung 49 der Patientenlagerungseinrichtung 1 gezeigt. Mit dem Finger seiner Hand 50 kann ein Benutzer Darstellungselemente manipulieren, um entsprechende Einstellungen an der Patientenlagerungseinrichtung 1 vorzunehmen. Vorliegend ist gemäß der Pfeile 51 die Trägereinrichtung 3 des mittleren Moduls angewählt.

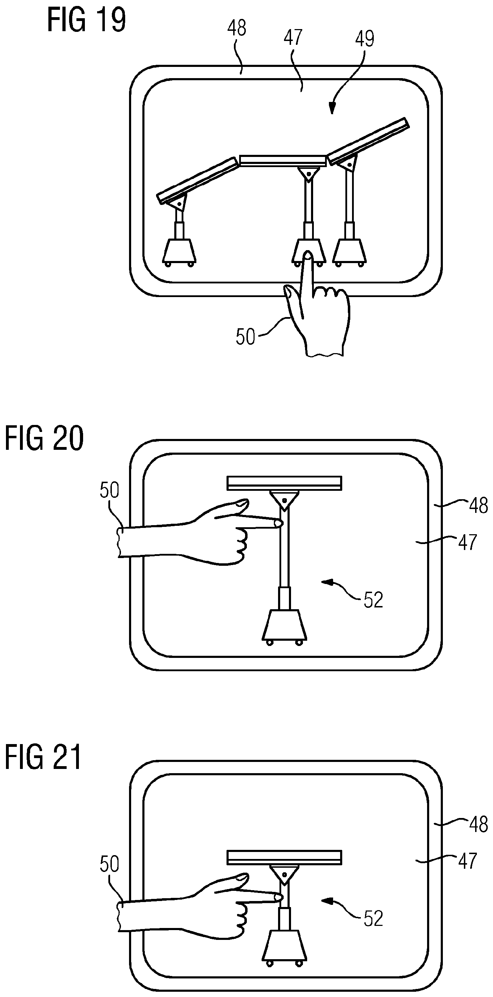

Fig. 19 zeigt die Situation zu einem späteren Zeitpunkt, wo zu erkennen ist, dass nun Dank der Aktoren der Längsverstellungseinrichtung und der Räder 9 beide Trägereinrichtungen 3 außen befindlich sind, so dass ein besonders großer Röntgenbildgebungsbereich 22 entsteht.

Die Figuren 20 und 21 zeigen die Bedienung eines einzelnen Moduls 2, von dem eine entsprechende Darstellung 52 gezeigt ist. Der Finger der Hand 50 manipuliert hier die Höhenverstellungseinrichtung des Moduls 2, was zu einer entsprechenden Höhenverstellung durch die Steuereinrichtung 29 führt.

Diese intuitive Bedienung zeigt nochmals deutlich, wie die Röntgendurchleuchtungsfähigkeit der jeweiligen als Operationstisch ausgebildeten Patientenlagerungseinrichtung 1, 6, 28, 35 den vorliegenden Anforderungen jederzeit angepasst werden kann, auch intraoperativ, nachdem, vgl. insbesondere auch die Figuren 18 und 19, die Aktoren so angesteuert werden können, dass sich die Positionierung des Patienten nicht verändert, aber die relative Position der Trägereinrichtungen 3. Wie die Ausführungsbeispiele auch gezeigt haben, leistet der hier dargestellte modulare Operationstisch eine Röntgentransparenz an beweglichen Stellen, also dort, wo die Teilplatten 4 aneinander treffen, an denen bei gewöhnlichen Operationstischen mechanische Komponenten, insbesondere das Schwenkgelenk 15, angeordnet ist.

Während in herkömmlichen Umsetzungen von verstellbaren Operationstischen viel Mechanik verbaut ist, um beispielsweise ein Knicken der Patientenlagerungsplatte 5 zu realisieren und um gleichzeitig die Stabilität des geknickten Operationstisches zu gewährleisten, ist die hier vorgesehene Montage von Teilplatten 4 auf zeitweise synchron arbeitenden und über die Steuereinrichtung 29 kommunizierenden Trägereinrichtungen 3 in der Lage, eine gelenklose Patientenlagerungsplatte 5 mit Knickfähigkeit zu gewährleisten, so dass der Patient 21 leichter für die diagnostische Bildgebungsverfahren, hier die Röntgenbildgebung, zugänglich ist.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Patientenlagerungseinrichtung (1, 6, 28, 35) für eine Röntgenbildgebungseinrichtung (23), aufweisend eine röntgentransparente Patientenlagerungsplatte (5) zur Lagerung eines Patienten (21) in einer Längsrichtung (8) und zumindest eine die Patientenlagerungsplatte (5) zumindest teilweise tragende Trägereinrichtung (3), wobei
- die Patientenlagerungsplatte (5) in wenigstens zwei Teilplatten (4) geteilt ist, wobei jede Teilplatte (4) an einer der Teilplatte (4) zugeordneten Trägereinrichtung (3) gelagert ist,
- jede Trägereinrichtung (3) wenigstens ein Schwenkgelenk zur Verschwenkung der Teilplatten (4) um eine horizontal, insbesondere senkrecht zur Längsrichtung (8), verlaufende Querachse (20) aufweist,
- -jede Trägereinrichtung (3) wenigstens eine Höhenverstelleinrichtung zur Höhenverstellung der Teilplatte (4) relativ zur Trägereinrichtung aufweist;
**dadurch gekennzeichnet, dass** jede Trägereinrichtung (3) eine Längsverstelleinrichtung zur Längsverschiebung der Teilplatte relativ zur Trägereinrichtung aufweist, dass die Trägereinrichtungen (3) mobil ausgebildet sind, und dass die Trägereinrichtungen mit ihren jeweiligen Teilplatten als voneinander unabhängige Module ausgebildet und flexibel einsetzbar sind, und diese zu unterschiedlichen konkreten Patientenlagerungseinrichtungen zusammenstellbar sind.

2. Patientenlagerungseinrichtung (1, 6, 28, 35) nach Anspruch 1, **dadurch gekennzeichnet, dass** jede Trägereinrichtung (3), insbesondere wenigstens ein Fuß (7) der Trägereinrichtung (3), derart massiv ausgebildet ist, dass bis in beide Endpositionen der Längsverstellung der Schwerpunkt oberhalb des Standbereichs der Trägereinrichtung (3) verbleibt, und/oder die Höhenverstelleinrichtung jeder insbesondere als Tragesäule ausgebildeten Trägereinrichtung (3) als eine Teleskopeinrichtung (11) ausgebildet ist und/oder die Längsverstelleinrichtung wenigstens eine Führungsschiene (18, 19) aufweist.

3. Patientenlagerungseinrichtung (1, 6, 28, 35) nach Anspruch 1 oder 2, **dadurch gekennzeichnet, dass** jedem Schwenkgelenk und/oder jeder Verstelleinrichtung ein ansteuerbarer Aktor zur jeweiligen Verstellung zugeordnet ist, wobei die Patientenlagerungseinrichtung (1, 6, 28, 35) ferner eine Steuereinrichtung (29) zur Ansteuerung aller Aktoren aufweist.

4. Patientenlagerungseinrichtung (1, 6, 28, 35) nach Anspruch 3, **dadurch gekennzeichnet, dass** die Steuereinrichtung (29) zur wenigstens teilweisen Kompensation eines durch Verschwenkung der Teilplatten (4) entstehenden Abstands zwischen den Teilplatten (4) durch Längsverschiebung und/oder Höhenverstellung der Teilplatten (4) ausgebildet ist.

5. Patientenlagerungseinrichtung (1, 6, 28, 35) nach Anspruch 3 oder 4, **dadurch gekennzeichnet, dass** die Steuereinrichtung (29) zum Empfang von Signalen einer der Patientenlagerungseinrichtung (1, 6, 28, 35) wenigstens zeitweise zugeordneten Bedieneinrichtung ausgebildet ist und/oder dass in der Steuereinrichtung (29) unterschiedlichen Operationsarten zugeordnete Grundstellungen gespeichert sind, welche bei benutzerseitiger Auswahl der entsprechenden Grundstellung angefahren werden.

6. Patientenlagerungseinrichtung (1, 6, 28, 35) nach Anspruch 5, **dadurch gekennzeichnet, dass** die Bedieneinrichtung (46) ein ein Touchdisplay (47) zur Anzeige einer manipulierbaren Darstellung (49, 52) der Patientenlagerungseinrichtung (1, 6, 28, 35) aufweisendes Mobilgerät, insbesondere ein Tablet (48), ist.

7. Patientenlagerungseinrichtung (1, 6, 28, 35) nach einem der Ansprüche 3 bis 6, **dadurch gekennzeichnet, dass** die Steuereinrichtung (29) zumindest bei in einer, insbesondere horizontalen, Ebene aneinander anschließenden Teilplatten (4) zur koordinierten Ansteuerung der Verstelleinrichtungen zur gemeinsamen Bewegung der gesamten Patientenlagerungsplatte (5) ausgebildet ist.

8. Patientenlagerungseinrichtung (1, 6, 28, 35) nach einem der vorangehenden Ansprüche, **dadurch gekennzeichnet, dass** die insbesondere röntgenopakes Material umfassenden Schwenkgelenke in Längsrichtung (8) nach außen in oder an der Trägereinrichtung (3) angeordnet sind.

9. Patientenlagerungseinrichtung (1, 6, 28, 35) nach einem der vorangehenden Ansprüche, **dadurch gekennzeichnet, dass** wenigstens zwei der wenigstens zwei benachbarten Teilplatten (4) auf ihrer einander zugewandten Seite durch ein, insbesondere lösbares und/oder flexibles und/oder dehnbares, Verbindungsmittel (30, 31, 37, 41) miteinander verbunden sind.

10. Patientenlagerungseinrichtung (1, 6, 28, 35) nach Anspruch 9, **dadurch gekennzeichnet, dass** an den jeweils einander zugewandten Oberflächen der Teilplatten (4) über elastische Befestigungsmittel (38) jeweils ein magnetischer Verbinder (39) befestigt ist.

11. Patientenlagerungseinrichtung (1, 6, 28, 35) nach einem der vorangehenden Ansprüche, **dadurch gekennzeichnet, dass** für wenigstens zwei der wenigstens zwei benachbarten Teilplatten (4) wenigstens eine der sich zugewandten Oberflächen der Teilplatten (4) und/oder Verbindungsmittel (30, 31, 37, 41) wenigstens einen in eine korrespondierende Führungsvertiefung (43) der anderen Oberfläche eingreifenden, eine Winkelstellung der Teilplatten (4) zueinander bei Eingriff erlaubenden Führungsvorsprung (42) aufweisen.

12. Patientenlagerungseinrichtung (1, 6, 28, 35) nach Anspruch 11, **dadurch gekennzeichnet, dass** der Führungsvorsprung (42) teilkugelförmig oder teilzylinderförmig ausgebildet ist und/oder wenigstens eine der Oberflächen senkrecht zur Längsrichtung (8) und zur Querrichtung (34) dem Führungsvorsprung (42) und/oder der Führungsvertiefung (43) benachbart zurückweichend und/oder Raum für eine Verkippung der Teilplatten (4) gegeneinander bereitstellend ausgebildet ist.

13. Patientenlagerungseinrichtung (1, 6, 28, 35) nach einem der vorangehenden Ansprüche, **dadurch gekennzeichnet, dass** die Patientenlagerungsplatte (5) mittig in zwei Teilplatten (4) geteilt ist.

14. Patientenlagerungseinrichtung (1, 6, 28, 35) nach einem der Ansprüche 1 bis 12, **dadurch gekennzeichnet, dass** sie wenigstens drei Teilplatten (4) aufweist und/oder das wenigstens zwei jeweils einem Bein (36) zugeordnete Teilplatten (4) in Querrichtung (34) aufeinander folgen.

15. Patientenlagerungseinrichtung (1, 6, 28, 35) nach einem der vorangehenden Ansprüche, **dadurch gekennzeichnet, dass** die Trägereinrichtungen (3) alle oder gruppenweise baugleich sind.

16. Patientenlagerungseinrichtung (1, 6, 28, 35) nach einem der vorangehenden Ansprüche, **dadurch gekennzeichnet, dass** die Trägereinrichtungen (3) Räder (9) aufweisen.

## Claims

1. Patient positioning device (1, 6, 28, 35) for an x-ray imaging device (23), comprising an x-ray-transparent patient positioning board (5) for positioning a patient (21) in a longitudinal direction (8), and at least one carrier device (3) at least partially supporting the patient positioning board (5), wherein
- the patient positioning board (5) is divided into at least two partial boards (4), wherein each partial board (4) is mounted on a carrier device (3) assigned to the partial board (4),
- each carrier device (3) has at least one pivot joint for pivoting the partial boards (4) about a transverse axis (20) which extends horizontally and in particular perpendicularly to the longitudinal direction (8),
- each carrier device (3) has at least one height adjustment device for height adjustment of the partial board (4) relative to the carrier device;
**characterised in that** each carrier device (3) has a longitudinal adjustment device for longitudinal displacement of the partial board relative to the carrier device, that the carrier devices (3) are designed to be mobile, and that the carrier devices are designed as modules which are independent of one another with their respective partial boards and can be inserted in a flexible manner, and these modules can be assembled with various specific patient positioning devices.

2. Patient positioning device (1, 6, 28, 35) according to claim 1, **characterised in that** each carrier device (3), in particular at least a foot (7) of the carrier device (3), is massively designed such that even at both end positions of the longitudinal adjustment the centre of gravity remains above the footprint of the carrier device (3), and/or the height adjustment device of each carrier device (3), designed as a support column in particular, is designed as a telescopic device (11), and/or the longitudinal adjustment has at least one guide rail (18, 19).

3. Patient positioning device (1, 6, 28, 35) according to claim 1 or 2, **characterised in that** each pivot joint and/or each adjustment device is assigned a triggerable actuator for respective adjustment, wherein the patient positioning device (1, 6, 28, 35) also has a control device (29) for triggering all actuators.

4. Patient positioning device (1, 6, 28, 35) according to claim 3, **characterised in that** the control device (29) is designed to compensate at least partially for a separation between the partial boards (4), caused by pivoting of the partial boards (4), by means of longitudinal displacement and/or height adjustment of the partial boards (4).

5. Patient positioning device (1, 6, 28, 35) according claim 3 or 4, **characterised in that** the control device (29) is designed to receive signals from an operating device which is assigned to a patient positioning device (1, 6, 28, 35) at least at certain times, and/or that basic arrangements assigned to different types of operation are stored in the control device (29) and then implemented when the corresponding basic arrangement is selected by a user.

6. Patient positioning device (1, 6, 28, 35) according to claim 5, **characterised in that** the operating device (46) is a mobile device, in particular a tablet (48), having a touch screen (47) for displaying a manipulatable representation (49, 52) of the patient positioning device (1, 6, 28, 35).

7. Patient positioning device (1, 6, 28, 35) according to one of claims 3 to 6, **characterised in that** in the case of partial boards (4) which abut each other in a plane, in particular a horizontal plane, the control device (29) is designed to trigger the adjustment devices in a coordinated manner in order to achieve overall movement of the complete patient positioning board (5).

8. Patient positioning device (1, 6, 28, 35) according to one of the preceding claims, **characterised in that** the pivot joints, which comprise x-ray-opaque material in particular, are arranged at the outside in a longitudinal direction (8), in or on the carrier device (3).

9. Patient positioning device (1, 6, 28, 35) according to one of the preceding claims, **characterised in that** at least two of the at least two adjacent partial boards (4) are interconnected on their facing sides by a connecting means (30, 31, 37, 41) which is in particular detachable and/or flexible and/or stretchable.

10. Patient positioning device (1, 6, 28, 35) according to claim 9, **characterised in that** a magnetic connector (39) is fastened in each case via elastic fastening means (38) to the respective facing surfaces of the partial boards (4).

11. Patient positioning device (1, 6, 28, 35) according to one of the preceding claims, **characterised in that** for at least two of the at least two adjacent partial boards (4), at least one of the facing surfaces of the partial boards (4) and/or connecting means (30, 31, 37, 41) has at least one guide projection (42) which engages into a corresponding guide recess (43) of the other surface and allows an angular arrangement of the partial boards (4) relative to each other when engaged.

12. Patient positioning device (1, 6, 28, 35) according to claim 11, **characterised in that** the guide projection (42) is partially spherical or partially cylindrical, and/or at least one of the surfaces perpendicular to the longitudinal direction (8) and to the transverse direction (34), and adjacent to the guide projection (42) and/or the guide recess (43), is so designed as to recede and/or allow room for the partial boards (4) to tilt relative to each other.

13. Patient positioning device (1, 6, 28, 35) according to one of the preceding claims, **characterised in that** the patient positioning board (5) is divided centrally into two partial boards (4).

14. Patient positioning device (1, 6, 28, 35) according to one of claims 1 to 12, **characterised in that** it has at least three partial boards (4) and/or that at least two partial boards (4) assigned in each case to a leg (36) are adjacently disposed in a transverse direction (34).

15. Patient positioning device (1, 6, 28, 35) according to one of the preceding claims, **characterised in that** all or groups of the carrier devices (3) are structurally identical.

16. Patient positioning device (1, 6, 28, 35) according to one of the preceding claims, **characterised in that** the carrier devices (3) have wheels (9).

## Revendications

1. Dispositif (1, 6, 28, 35) de mise en position de patient pour un dispositif (23) d'imagerie par rayons X, comportant un plateau (5) transparent aux rayons X de mise en position du patient pour la mise en position d'un patient (21) dans une direction (8) longitudinale et au moins un dispositif (3) de support supportant au moins en partie le plateau (5) de mise en position du patient, dans lequel
- le plateau (5) de mise en position du patient est subdivisé en au moins deux plateaux (4) partiels, dans lequel chaque plateau (4) partiel est monté sur un dispositif (3) de support affecté à l'un des plateaux (4) partiels,
- chaque dispositif (3) de support a au moins une articulation de pivotement pour le pivotement des plateaux (4) partiels autour d'un axe (20) transversal s'étendant horizontalement, en particulier perpendiculairement à la direction (8) longitudinale,
- chaque dispositif (3) de support a au moins un dispositif de réglage en hauteur pour le réglage en hauteur du plateau (4) partiel par rapport au dispositif de support ;
**caractérisé en ce que** chaque dispositif (3) de support a un dispositif de réglage en longueur pour le décalage en longueur du plateau partiel par rapport au dispositif de support, **en ce que** les dispositifs (3) de support sont de constitution mobile, et **en ce que** les dispositifs de support sont constitués, par leurs plateaux mobiles respectifs, sous la forme de modules indépendants les uns des autres et peuvent être utilisés de manière souple, et ceux-ci peuvent être rassemblés en des dispositifs concrets différents de mise en position de patient.

2. Dispositif (1, 6, 28, 35) de mise en position de patient suivant la revendication 1, **caractérisé en ce que** chaque dispositif (3) de support, en particulier au moins un pied (7) du dispositif (3) de support, est de constitution pleine, de manière à laisser subsister, jusque dans les deux positions d'extrémité du réglage en longueur, le centre de gravité au-dessus de la partie érigée du dispositif (3) de support, et/ou le dispositif de réglage en hauteur de chaque dispositif (3) de support, constitué en particulier sous la forme d'une colonne porteuse, est constitué sous la forme d'un dispositif (11) télescopique et/ou le dispositif de réglage en longueur a au moins un rail (18, 19) de guidage.

3. Dispositif (1, 6, 28, 35) de mise en position de patient suivant l'une des revendications 1 ou 2, **caractérisé en ce que**, à chaque articulation de pivotement et/ou à chaque dispositif de réglage, est associé un actionneur pouvant être commandé pour le réglage respectif, dans lequel le dispositif (1, 6, 28, 35) de mise en position de patient a en outre un dispositif (29) de commande pour la commande de tous les actionneurs.

4. Dispositif (1, 6, 28, 35) de mise en position de patient suivant la revendication 3, **caractérisé en ce que** le dispositif (29) de commande est constitué pour la compensation au moins de temps à autre d'une distance se créant par pivotement des plateaux (4) partiels entre les plateaux (4) partiels par décalage longitudinal et/ou réglage en hauteur des plateaux (4) partiels.

5. Dispositif (1, 6, 28, 35) de mise en position de patient suivant la revendication 3 ou 4, **caractérisé en ce que** le dispositif (29) de commande est constitué pour la réception de signaux d'un dispositif de service associé au moins de temps en temps au dispositif (1, 6, 28, 35) de mise en position de patient et/ou **en ce que**, dans le dispositif (29) de commande, sont mises en mémoire des positions de base associées à des types de fonctionnements différents, qui sont mises en service lors du choix par l'utilisateur de la position de base correspondante.

6. Dispositif (1, 6, 28, 35) de mise en position de patient suivant la revendication 5, **caractérisé en ce que** le dispositif (46) de service est un appareil mobile, en particulier une tablette (48) ayant un écran (47) à touches, pour l'affichage d'une représentation (49, 52) manipulable du dispositif (1, 6, 28, 35) de mise en position du patient.

7. Dispositif (1, 6, 28, 35) de mise en position de patient suivant l'une des revendications 3 à 6, **caractérisé en ce que** le dispositif (29) de commande est constitué pour la commande coordonnée au moins dans un plan, en particulier horizontal, de plateaux (4) partiels se raccordant l'un à l'autre, des dispositifs de réglage pour le déplacement conjoint de l'ensemble du plateau (5) de mise en position du patient.

8. Dispositif (1, 6, 28, 35) de mise en position de patient suivant l'une des revendications précédentes, **caractérisé en ce que** des articulations de pivotement, comprenant en particulier du matériau opaque aux rayons X, sont disposées dans la direction (8) longitudinale vers l'extérieur dans ou sur le dispositif (3) de support.

9. Dispositif (1, 6, 28, 35) de mise en position de patient suivant l'une des revendications précédentes, **caractérisé en ce qu'**au moins deux des au moins deux plateaux (4) partiels voisins sont, sur leurs côtés tournés l'un vers l'autre, assemblés l'un à l'autre par un moyen (30, 31, 37, 41) d'assemblage, en particulier pouvant être défait et/ou souple et/ou extensible.

10. Dispositif (1, 6, 28, 35) de mise en position de patient suivant la revendication 9, **caractérisé en ce que**, sur les surfaces tournées l'une vers l'autre respectivement des plateaux (4) partiels, respectivement un connecteur (39) magnétique est fixé par des moyens (38) de fixation élastiques.

11. Dispositif (1, 6, 28, 35) de mise en position de patient suivant l'une des revendications précédentes, **caractérisé en ce que**, pour au moins deux des au moins deux plateaux (4) partiels voisins, au moins l'une des surfaces tournées l'une vers l'autre des plateaux (4) partiels et/ou des moyens (30, 31, 37, 41) de liaison ont au moins une saillie (42) de guidage pénétrant dans une cavité (43) de guidage correspondante de l'autre surface et permettant, lors de la pénétration l'une par rapport à l'autre, un réglage angulaire des plateaux (4) partiels.

12. Dispositif (1, 6, 28, 35) de mise en position de patient suivant la revendication 11, **caractérisé en ce que** la saillie (42) de guidage est en forme de sphère partielle ou en forme de cylindre partiel et/ou au moins l'une des surfaces est constituée pour le retour au voisinage de la saillie (42) de guidage et/ou la cavité (43) de guidage perpendiculairement à la direction (8) longitudinale et à la direction (34) transversale et/ou pour ménager un espace pour un basculement des plateaux (4) partiels l'un par rapport à l'autre.

13. Dispositif (1, 6, 28, 35) de mise en position de patient suivant l'une des revendications précédentes, **caractérisé en ce que** le plateau (5) de mise en position du patient est subdivisé au milieu en deux plateaux (4) partiels.

14. Dispositif (1, 6, 28, 35) de mise en position de patient suivant l'une des revendications 1 à 12, **caractérisé en ce qu'**il a au moins trois plateaux (4) partiels et/ou les au moins deux plateaux (4) partiels associés respectivement à une jambe (36) se suivent l'un l'autre dans la direction (34) transversale.

15. Dispositif (1, 6, 28, 35) de mise en position de patient suivant l'une des revendications précédentes, **caractérisé en ce que** les dispositifs (3) de support sont tous ou groupe par groupe de même construction.

16. Dispositif (1, 6, 28, 35) de mise en position de patient suivant l'une des revendications précédentes, **caractérisé en ce que** les dispositifs (3) de support ont des roulettes (9).
